# EUROPEAN PATENT SPECIFICATION

(11) **EP 2 917 344 B1**
(45) Date of publication and mention of the grant of the patent: **22.08.2018**
(21) Application number: 13786287.6
(22) Date of filing: 06.11.2013
(51) Int. Cl.: C12N 15/10, C12Q 1/6806

(54) **METHODS FOR ONE STEP NUCLEIC ACID AMPLIFICATION OF NON-ELUTED SAMPLES**
VERFAHREN ZUR EINSTUFIGEN NUKLEINSÄUREAMPLIFIKATION VON NICHT-ELUIERTEN PROBEN
MÉTHODES D'AMPLIFICATION D'ACIDES NUCLÉIQUES EN UNE ÉTAPE D'ÉCHANTILLONS NON ÉLUÉS

(30) Priority: 09.11.2012 GB 201220240; 25.01.2013 GB 201301344; 15.03.2013 US 201361789788 P
(43) Date of publication of application: 16.09.2015
(73) Proprietor: GE Healthcare UK Limited, Little Chalfont, Buckinghamshire HP7 9NA (GB)
(72) Inventor: TATNELL, Peter James, Cardiff South Glamorgan CF14 7YT (GB); LAMERTON, Kathryn, Louise, Cardiff South Glamorgan CF14 7YT (GB); PIERCE, Alan, Stuart, Cardiff South Glamorgan CF14 7YT (GB); ASHMAN, Elizabeth, Cardiff South Glamorgan CF14 7YT (GB)
(74) Representative: Bannan, Sally
(86) International application number: PCT/EP2013/073189
(87) International publication number: WO 2014/072354

(56) References cited:
- WO-A1-96/39813
- WO-A1-96/39813
- US-A- 5 756 126
- US-A- 5 939 259
- US-A1- 2004 101 895
- CARLA R. SANTOS ET AL: "Use of FTA elute card impregnated with cervicovaginal sample directly into the amplification reaction increases the detection of human papillomavirus DNA", BRAZILIAN JOURNAL OF MICROBIOLOGY, vol. 43, no. 1, 1 January 2012 (2012-01-01), pages 389-392, XP055089528, ISSN: 1517-8382, DOI: 10.1590/S1517-83822012000100047
- Pak Yang Chum ET AL: "Direct PCR from blood preserved on Whatman FTA and 903 Cards using Phusion Blood Direct PCR Kit", , 1 December 2008 (2008-12-01), pages 1-1, XP055057288, Retrieved from the Internet: URL:http://www.thermofisher.com.au/Uploads /file/Scientific/Applications/Life-Science -Research-Technologies/Direct-PCR-from-blo od-preserved-on-Whatman-FTA-and-903-Cards. pdf [retrieved on 2013-03-21]
- MICHAUD ET AL: "Long-term storage at tropical temperature of dried-blood filter papers for detection and genotyping of RNA and DNA viruses by direct PCR", JOURNAL OF VIROLOGICAL METHODS, ELSEVIER BV, NL, vol. 146, no. 1-2, 3 November 2007 (2007-11-03), pages 257-265, XP022327162, ISSN: 0166-0934, DOI: 10.1016/J.JVIROMET.2007.07.006
- BRIAN J TAYLOR ET AL: "Real-time PCR detection of Plasmodium directly from whole blood and filter paper samples", MALARIA JOURNAL, BIOMED CENTRAL, LONDON, GB, vol. 10, no. 1, 19 August 2011 (2011-08-19), page 244, XP021092124, ISSN: 1475-2875, DOI: 10.1186/1475-2875-10-244

## Description

### Filed of Invention

The present invention relates to the field of nucleic acid amplification, particularly to the use of a polymerase chain reaction to amplify nucleic acids. The invention provides methods and kits which can be used to amplify nucleic acids by combining an FTA™ Elute solid support with PCR reagents for one step amplification of nucleic acid samples. The invention has applications in the long term storage and easy processing of nucleic acids and is particularly useful in genotyping, diagnostics and forensics.

### Background

The polymerase chain reaction (PCR) is a common tool used in molecular biology for amplifying nucleic acids. US4683202 (Mullis, Cetus Corporation) describes a process for amplifying any desired specific nucleic acid sequence contained in a nucleic acid or mixture thereof.

Long-term storage, transport and archiving of nucleic acids on filter paper or chemically modified matrices is a well-known technique for preserving genetic material before the DNA or RNA is extracted and isolated in a form for use in genetic analysis such as PCR. Thus, EP1563091 (Smith *et al,* Whatman) relates to methods for storing nucleic acids from samples such as cells or cell lysates. The nucleic acid is isolated and stored for extended periods of time, at room temperature and humidity, on a wide variety of filters and other types of solid support or solid phase media. Moreover, the document describes methods for storing nucleic acid-containing samples on a wide range of solid support matrices in tubes, columns, or multiwell plates.

Taylor et al. (2011 Malaria J; 10: 244) teach real time PCR directly from whole blood using Whatman 3mm filter paper where the paper does not contain any chemicals. A disc of the filter paper containing the sample is used in a direct PCR reaction.

WO/9003959 (Burgoyne) describes a cellulose-based solid support for the storage of DNA, including blood DNA, comprising a solid matrix having a compound or composition which protects against degradation of DNA incorporated into or absorbed on the matrix. This document also discloses methods for storage of DNA using the solid medium, and for recovery of or *in situ* use of DNA.

US5496562 (Burgoyne) describes a cellulose-based solid medium and method for DNA storage. Method for storage and transport of DNA on the solid medium, as well as methods which involve either (a) the recovery of the DNA from the solid medium or (b) the use of the DNA *in situ* on the solid medium (for example, DNA sequence amplification by PCR) are disclosed. Unfortunately, the methods described only incorporates a surfactant or detergent on the surface of the solid medium and therefore suffer from the disadvantage that they require a separate step for the removal of the detergent before PCR is performed.

WO993900 (Gentra) describes again a method for processing and amplifying DNA. The method includes the steps of contacting the sample containing DNA to a solid support wherein a lysis reagent is bound to the solid support. The DNA is subsequently treated with a DNA purifying reagent and is purified. The application does not include a sequestrant on the solid support and requires a separate step for the removal of the lysis reagent and purification of the DNA before amplification.

WO9639813 (Burgoyne) describes a solid medium for storing a sample of genetic material and subsequent analysis; the solid medium comprising a protein denaturing agent and a chelating agent. The method described is for chelating agents which are any compound capable of complexing multivalent ions including Group II and Group III multivalent metal ions and transition metal ions. The invention does not specifically mention cyclodextrin as a chelating agent, nor does it suggest the PCR analysis could be performed in a single step.

US5705345 (Lundin et al.) describes a method of nucleic acid preparation whereby the sample containing cells is lysed to release nucleic acid and the sample is treated with cyclodextrin to neutralize the extractant. The advantage of this system is that conventional detergent removal requires a separation step however with the addition of cyclodextrin to neutralize the detergent it would remove the separation step needed and reduce chance of contamination.

GB2346370 (Cambridge Molecular Technologies Ltd) describes applying a sample comprising cells containing nucleic acid to a filter, the cells are retained by the filter and contaminants are not. The cells are lysed on the filter and retained alongside the nucleic acid. Subsequent steps filter out the cell lysate while retaining the nucleic acid.

WO9618731 (Deggerdal) describes a method of isolating nucleic acid whereby the sample is bound to a solid support and sample is contacted with a detergent and subsequent steps performed to isolate the nucleic acid.

WO0053807 (Smith, Whatman) describes a medium for the storage and lysis of samples containing genetic material which can be eluted and analysed. The medium is coated with a lysis reagent. In addition the medium could be coated with a weak base, a chelating agent, a surfactant and optionally uric acid.

WO9938962 (Health, Gentra Systems Inc.) describes a solid support with a bound lysis reagent. The lysis reagent can comprise of a detergent, a chelating agent, water and optionally an RNA digesting enzyme. The solid support does not contain cyclodextrin and requires further steps for purification of the nucleic acid for amplification analysis.

Current methods for DNA amplification involve a DNA purification procedure which often involves several steps which increases the chance of contamination. This is a tedious process and prior art methods have a number of clear disadvantages in terms of cost, complexity and in particular, user time. For example, column-based nucleic acid purification is a typical solid phase extraction method to purify nucleic acids. This method relies on the nucleic acid binding through adsorption to silica or other support depending on the pH and the salt content of the buffer. Examples of suitable buffers include Tris-EDTA (TE) buffer or Phosphate buffer (used in DNA microarray experiments due to the reactive amines). The purification of nucleic acids on such spin columns includes a number of complex and tedious steps. Nucleic acid purification on spin columns typically involves three time-consuming and complex steps/stages:
the sample containing nucleic acid is added to the column and the nucleic acid binds due to the lower pH (relative to the silanol groups on the column) and salt concentration of the binding solution, which may contain buffer, a denaturing agent (such as guanidine hydrochloride), Triton X-100, isopropanol and a pH indicator;
the column is washed with 5 mM KPO4 pH 8.0 or similar, 80% EtOH); and the column is eluted with buffer or water.

Alternative methods involve the binding of nucleic acids in the presence of chaotropic salts such that DNA binds to silica or glass particles or glass beads. This property was used to purify nucleic acid using glass powder or silica beads under alkaline conditions. Typical chaotropic salts include guanidinium thiocyanate or guanidinium hydrochloride and recently glass beads have been substituted with glass containing minicolumns.

The best defence against PCR amplification failure in forensics applications is to combine sound sample handling and processing techniques with extraction systems proven to efficiently purify DNA.

Santos C.R. et al., (Brazilian Journal of Microbiology, 2012, 43, 389-392) describes a method of skipping the elution step prior to PCR amplification of nucleic acid and adding the punchers directly into the PCR mix. The PCR amplification was performed for the detection of HPV-DNA and was more efficient than the standard FTA elute card protocol of eluting the nucleic acid prior to amplification. However this method only used qualitative PCR to measure the presence of HPV.

Nozawa N. et al., (Journal of Clinical Microbiology, 2007, 45, 1305-1307) describes a method of using real time PCR for the detection of cytomegalovirus (CMV). The method described involved the use of filter paper with purified CMV and was added directly to the PCR mix. The paper noted that only instruments with a photo-multiplier-tube scanning system could be used for real time PCR assays with filter disks. The paper suggests that the filter paper would adversely affect instruments using a charge-coupled device camera and therefore teaches away from the use of filter papers in real time PCR machines such as an ABI7700 machine.

Qiagen Sample & Assay Technologies Newsletter (March 2010, 15) describes the effects of a low A₂₆₀/A₂₃₀ ratio in RNA preparations on downstream PCR processing. The newsletter notes that increased absorbance at 230nm in RNA samples is quite often due to contamination with guanidine thiocynate (a component of FTA elute cards and is used in RNA purification procedures). The experiments demonstrate A₂₆₀/A₂₃₀ ratio of an RNA sample is lower when guanidine thiocyanate is present, however guanidine thiocyanate concentrations up to 100mM in an RNA sample did not affect the reliability of real-time PCR.

Chum et al (2008 "Direct PCR from blood preserved on Whatman FTA and 903 Cards using Phusion® Blood Direct PCR Kit") recognise that FTA elute will inhibit PCR, however they use an engineered polymerase resistant to PCR inhibitors that works in the presence of inhibitory chemicals present in the cards.

Typically the purification steps involved in the standard FTA elute card protocol can be cumbersome and purification can lead to a loss in DNA. There is therefore a need for an improved and simplified process for amplifying, quantifying and or profiling nucleic acid, which removes the need for a purification step. The present invention addresses this problem and provides methods and kits which can be used for single step amplification of nucleic acid from solid supports, particularly cellulose-derived supports.

### Summary of Invention

The present invention provides methods and kits which can be used to amplify nucleic acids by contacting a solid support with nucleic acid and amplifying the nucleic acid in the presence of said solid support for easy amplification of DNA samples.

According to a first aspect of the present invention, there is provided a method for amplification of nucleic acid comprising the steps:
i) contacting a solid support comprising a chaotropic salt with a cellular sample containing nucleic acid,
ii) transferring said solid support to a reaction vessel,
iii) incubating said nucleic acid on the solid support with a nucleic acid amplification reagent solution,
iv) amplifying the nucleic acid to produce amplified nucleic acid wherein the method of amplification is a quantitative polymerase chain reaction,
v) quantifying the amplified nucleic acid and optionally,
vi) using Short Tandem Repeat (STR) profiling to produce an STR profile,
wherein steps i) to vi) are carried out in the presence of the solid support.

The advantage of amplifying the nucleic acid in the presence of the solid support is to reduce the number of steps required for nucleic acid amplification, thus saving operator time and facilitating operator usage.

In one aspect of the present invention, wherein the solid support is already in the reaction vessel prior to the addition of said cellular sample.

In a further aspect, the nucleic acid amplification reagent solution comprises a polymerase, deoxyribonucleotide triphosphate (dNTP), a reaction buffer and at least one primer, wherein said primer is optionally labeled with a dye. Such dyes may include fluorescence dye FAM™ or CyDye DIGE Fluor™ from GE Healthcare (product code RPK0272). The nucleic acid amplification reagent solution can be present in a dried form, such as a "Ready-to-Go™" (RTG) format. The advantage of dried or lyophilised formulations of the polymerase chain reaction reagents is that they can be easily
solublised by the addition of water, thus saving operator time and facilitating operator usage. To minimise operator error, the dried reagent mixture can be pre-dispensed into the reaction vessel, such as the well of a multi-well plate. Examples of such an RTG mixture include "Illustra Ready-to-Go RT-PCR beads" available from GE Healthcare (product code: 27-9266-01 Illustra Ready-To-Go RT-PCR Beads).

In a further aspect, the STR profile reagents are selected from the group consisting of PowerPlex 18D, PowerPlex 21, PowerPlex Fusion, Identifier Direct, Globalfiler Express and Y-Filer Direct.

In a further aspect, the nucleic acid is selected from the group consisting of DNA, RNA and oligonucleotide. The term "nucleic acid" is used herein synonymously with the term "nucleotides" and includes DNA, such as plasmid DNA and genomic DNA; RNA, such as mRNA, tRNA, sRNA and RNAi; and protein nucleic acid, PNA.

In one aspect, the chaotropic salt is a guanidine salt.

In another aspect, said guanidine salt is selected from the group consisting of guanidine thiocyanate, guanidine chloride and guanidine hydrochloride.

In one aspect, the chaotropic salt is sodium salt such as sodium iodide.

In another aspect, the solid support is washed with an aqueous solution following step i).

In one aspect, the solid support is selected from the group consisting of a glass or silica-based solid phase medium, a plastics-based solid phase medium, a cellulose-based solid phase medium, glass fiber, glass microfiber, silica gel, silica oxide, nitrocellulose, carboxymethylcellulose, polyester, polyamide, carbohydrate polymers, polypropylene, polytetraflurorethylene, polyvinylidinefluoride, wool and porous ceramics.

In another aspect, the solid support is a cellulose based matrix.

In a further aspect, said cellulose based matrix is in the form of a pre punched disc.

In another aspect, said cellulose based matrix is in the form of an FTA™ Elute card.

In another aspect, said cellulose based matrix is in the form of an indicating FTA™ Elute (iFTAe) Card wherein the dye indicates the presence of a biological sample.

In one aspect, wherein the amplified nucleic acid is quantified using a PCR imaging system.

In one aspect the cellular sample is selected from a group consisting of eukaryotic or prokaryotic cell, virus, bacteria, plant and tissue culture cells.

In another aspect, said cellular sample is selected from the group consisting of blood, serum, semen, cerebral spinal fluid, synovial fluid, lymphatic fluid, saliva, buccal, cervical cell, vaginal cell, urine, faeces, hair, skin and muscle. The cellular sample may originate from a mammal, bird, fish or plant or a cell culture thereof. Preferably the cellular sample is mammalian in origin, most preferably human in origin. The sample containing the nucleic acid may be derived from any source. This includes, for example, physiological/pathological body fluids (e.g. secretions, excretions, exudates) or cell suspensions of humans and animals; physiological/pathological liquids or cell suspensions of plants; liquid products, extracts or suspensions of bacteria, fungi, plasmids, viruses, prions, etc.; liquid extracts or homogenates of human or animal body tissues (e.g., bone, liver, kidney, etc.); media from DNA or RNA synthesis, mixtures of chemically or biochemically synthesized DNA or RNA; and any other source in which DNA or RNA is or can be in a liquid medium.

In a further aspect, the method is for use as a tool selected from the group consisting of a molecular diagnostics tool, a human identification tool, a forensics tool, STR profiling tool and DNA profiling.

In another aspect, wherein the nucleic acid is stored on the solid support prior to step ii).

In one aspect, the nucleic acid is stored on the solid support for at least 30 minute. The nucleic acid may be immobilised on the solid support for longer periods, for example, for at least 24 hours, for at least 7 days, for at least 30 days, for at least 90 days, for at least 180 days, for at least one year, and for at least 10 years. In this way the nucleic acid may be stored in a dried form which is suitable for subsequent analysis. Typically, samples are stored at temperatures from -200°C to 40°C. In addition, stored samples may be optionally stored in dry or desiccated conditions or under inert atmospheres.

The method of the invention can be used either in single tube or a high-throughput 96-well format in combination with automated sample processing as described by Baron et al., (2011, Forensics Science International: Genetics Supplement Series, 93, e560-e561). This approach would involve a minimal number of steps and increase sample throughput. The risk of operator-induced error, such as cross-contamination is also reduced since this procedure requires fewer manipulations compared to protocols associated with currently used, more labour intensive kits (e.g. QIAmp DNA blood mini kit, Qiagen). The risk of sample mix-up is also reduced since the procedure requires few manipulations. Importantly, the method is readily transferable to a multi-well format for high-throughput screening. The present invention can thus improve sample processing for carrying out PCR reactions to aid genetic interrogations. The invention can be conducted in a 96 well/high throughput format to facilitate sample handling and thus eliminate batch processing of samples.

In a further aspect, the reaction vessel is a well in a multi-well plate. Multi-well plates are available in a variety of formats, including 6, 12, 24, 96, 384 wells (e.g. Corning 384 well multi-well plate, Sigma Aldrich).

In one aspect, the sample is transferred to the reaction vessel by punching or cutting a disc from the solid support. Punching the portion or disc from the solid support can be effected by use of a punch, such as a Harris Micro Punch (Whatman Inc.; Sigma Aldrich)

According to a second aspect of the present invention there is provided a method for amplification of nucleic acid comprising the steps:
i) contacting a solid support comprising a chaotropic salt with a cellular sample containing nucleic acid,
ii) transferring said solid support to a reaction vessel,
iii) incubating said nucleic acid on the solid support with a nucleic acid amplification reagent solution,
iv) amplifying the nucleic acid to produce amplified nucleic acid,
v) quantifying the amplified nucleic acid wherein the amplified nucleic acid is quantified using a PCR imaging system,
wherein steps i) to v) are carried out in the presence of the solid support.

In one aspect, wherein the solid support is already in the reaction vessel prior to the addition of said cellular sample.

In another aspect, wherein the method of amplification is a polymerase chain reaction.

In another aspect, the chaotropic salt is selected from the group consisting of, guanidine thiocynate, guanidine chloride, guanidine hydrochloride and sodium iodide.

In a further aspect, said solid support is impregnated with sodium dodecyl sulfate (SDS), ethylenediaminetetracetic acid (EDTA) and uric acid.

In one aspect, said solid support is in the form of an FTA™ pre punched disc.

In another aspect, said cellulose based matrix is in the form of an indicating FTA™ (iFTA) Card wherein the dye indicates the presence of a biological sample.

In another aspect, the solid support is selected from the group consisting of a glass or silica-based solid phase medium, a plastics-based solid phase medium or a cellulose-based solid phase medium, glass fiber, glass microfiber, silica gel, silica oxide, nitrocellulose, carboxymethylcellulose, polyester, polyamide, carbohydrate polymers, polypropylene, polytetraflurorethylene, polyvinylidinefluoride, wool or porous ceramics.

In another aspect, the solid support is washed with an aqueous solution following step i).

In one aspect, wherein the cellular sample is selected from a group consisting of eukaryotic or prokaryotic cell, virus, bacteria, plant and tissue culture cells.

In another aspect, said cellular sample is selected from the group consisting of blood, serum, semen, cerebral spinal fluid, synovial fluid, lymphatic fluid, saliva, buccal, cervical and vaginal cells, urine, faeces, hair, skin and muscle. The cellular sample may originate from a mammal, bird, fish or plant or a cell culture thereof. Preferably the cellular sample is mammalian in origin, most preferably human in origin. The sample containing the nucleic acid may be derived from any source. This includes, for example, physiological/pathological body fluids (e.g. secretions, excretions, exudates) or cell suspensions of humans and animals; physiological/pathological liquids or cell suspensions of plants; liquid products, extracts or suspensions of bacteria, fungi, plasmids, viruses, prions, etc.; liquid extracts or homogenates of human or animal body tissues (e.g., bone, liver, kidney, etc.); media from DNA or RNA synthesis, mixtures of chemically or biochemically synthesized DNA or RNA; and any other source in which DNA or RNA is or can be in a liquid medium.

In a further aspect, the method is for use as a tool selected from the group consisting of a molecular diagnostics tool, a human identification tool, a forensics tool, STR profiling tool and DNA profiling.

In another aspect, wherein the nucleic acid is stored on the solid support prior to step ii).

In one aspect, the nucleic acid is stored on the solid support for at least 30 minute. The nucleic acid may be immobilised on the solid support for longer periods, for example, for at least 24 hours, for at least 7 days, for at least 30 days, for at least 90 days, for at least 180 days, for at least one year, and for at least 10 years. In this way the nucleic acid may be stored in a dried form which is suitable for subsequent analysis. Typically, samples are stored at temperatures from -200°C to 40°C. In addition, stored samples may be optionally stored in dry or desiccated conditions or under inert atmospheres.

The method of the invention can be used either in single tube or a high-throughput 96-well format in combination with automated sample processing as described by Baron et al., (2011, Forensics Science International: Genetics Supplement Series, 93, e560-e561). This approach would involve a minimal number of steps and increase sample throughput. The risk of operator-induced error, such as cross-contamination is also reduced since this procedure requires fewer manipulations compared to protocols associated with currently used, more labour intensive kits (e.g. QIAmp DNA blood mini kit, Qiagen). The risk of sample mix-up is also reduced since the procedure requires few manipulations. Importantly, the method is readily transferable to a multi-well format for high-throughput screening. The present invention can thus improve sample processing for carrying out PCR reactions to aid genetic interrogations. The invention can be conducted in a 96 well/high throughput format to facilitate sample handling and thus eliminate batch processing of samples.

In a further aspect, the reaction vessel is a well in a multi-well plate. Multi-well plates are available in a variety of formats, including 6, 12, 24, 96, 384 wells (e.g. Corning 384 well multi-well plate, Sigma Aldrich).

In one aspect, the sample is transferred to the reaction vessel by punching or cutting a disc from the solid support. Punching the portion or disc from the solid support can be effected by use of a punch, such as a Harris Micro Punch (Whatman Inc.; Sigma Aldrich)

According to a third aspect of the present invention there is provided a kit for amplifying nucleic acid as herein before described and instructions for use thereof.

### Brief Description of the Figures

Figure 1 Presents the STR profile from the PCR amplification of unwashed HeLa cell spotted iFTAe (replicate no. 1).
Figure 2 Presents the STR profile from the PCR amplification of unwashed HeLa cell spotted iFTAe (replicate no. 2).
Figure 3 Presents the STR profile from the PCR amplification of unwashed HeLa cell spotted iFTAe (replicate no. 3).
Figure 4 Presents the STR profile from the PCR amplification of control DNA sample.
Figure 5 Presents the DNA yield from the qPCR amplification of washed blood spotted iFTAe amplified directly.
Figure 6 Presents the DNA yield from the qPCR amplification of unwashed HeLa cell spotted iFTAe either amplified directly or after being eluted.

### Detailed Description of the Invention

### Chemicals and Materials Used

A list of the chemicals and their sources is given below:
Indicating FTA™ elute micro (WB120218 and WB120411);
Indicating FTA™ elute cassette (WB120230);
Normal human blood (Tissue Solutions Ltd);
Genomic DNA (Promega product code G152A);
Harris Uni-core punch, 1.2mm (Sigma, Catalogue number Z708860-25ea, lot 3110);
TaqMan Universal PCR master Mix, no AmpErase UNG (Applied Biosystems part number 4324018);
TaqMan RNase P Detection Reagents (Applied Biosystems part number 4316831) - contains RNase P primer;
PowerPlex 18D (Promega code DC1802) - contains primers;
Sterile water (Sigma Product code W4502);
Huma cervical epithelial cells (HeLa) (ATCC code CCL-2) and
Hi-Di Formamide (ABI code 4311320)

### Experimental Results

### STR Profiles from Hela cell spotted Elute FTA cards

Cultured HeLa cells at a concentration of 2.5x10⁶ cell/ml were spotted onto an indicating FTA elute (iFTAe) card. A 1.2mm punch was taken from the cell spotted FTA elute card and combined with a direct STR kit PowerPlex 18D reaction mix for a final volume of 25µl. The 25µl sample mix was added to each well of a 96 well PCR plate prior to amplification. Samples were analysed on a 3130xl Capillary Electrophoresis using a 10 second sample injection.

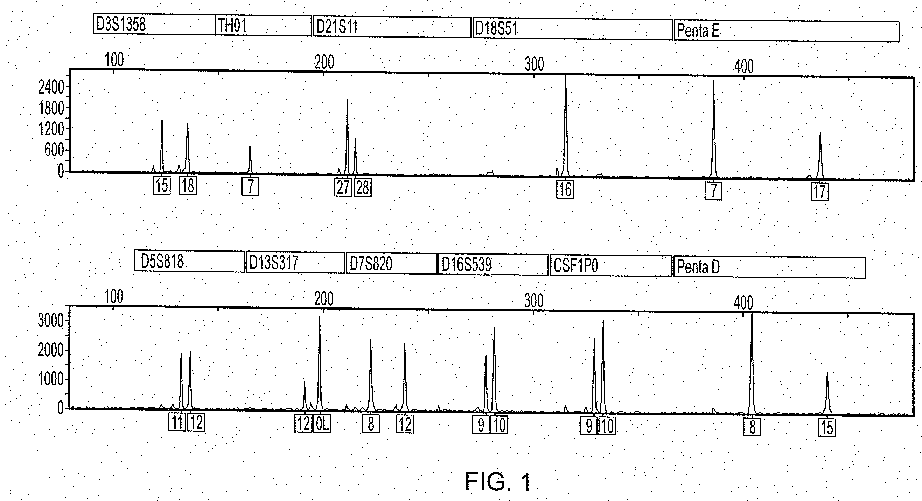

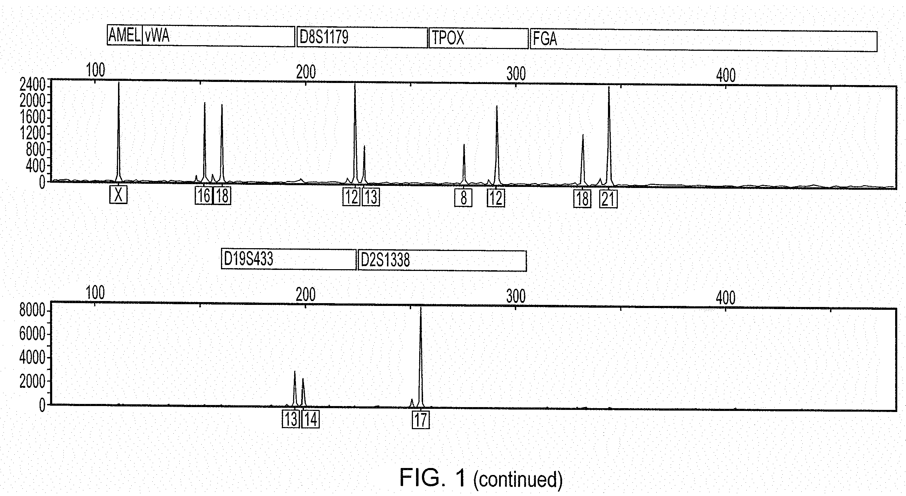

PCR reaction was set up as follows:
Standards and samples were added to the appropriate wells. The plates were sealed and centrifuged at 1000rpm for 1 minute. PCR was carried out on a Geneamp/ABI 9700 Thermo Cycler under the following thermal cycling conditions:
96°C for 2min, followed by 28 cycles of: 94°C for 10sec, 60°C for 1min, followed by 60°C for 20min, followed by a 4°C. Following amplification, visualisation of PCR products was achieved using Capillary Electrophoresis. The results are presented graphically in Figure 1 to 4.

**Table 1: Volume of reagents in HeLa cell reaction mix**

| **Ingredients** | **Volume** |
|---|---|
| High Grade Water | 15µl |
| Primers | 5µl |
| Reaction mix | 5µl |
| 1.2mm punch of FTA elute containing HeLa cells. | 1 punch |

**Table 2: Volume of reagents in DNA control reaction mix**

| **Ingredients** | **Volume** |
|---|---|
| High Grade Water | 15µl |
| Primers | 5µl |
| Reaction mix | 5µl |
| 2800M control DNA sample (5ng/µl) | 1µl |

**Table 3: Volumes of reagents used in the Capillary Electrophoresis**

| **Ingredients** | **Volume** |
|---|---|
| Hi-Di Formamide | 10µl |
| CC5 Internal Lane Standard | 1µl |
| PCR amplified cell sample or control DNA sample | 1µl |
| Total volume | 12µl |

Figure 1 shows STR profile of unwashed HeLa cell spotted indicating FTA elute card combined with STR PCR reagents (replicate 1). The average peak height was 2313 RFU.

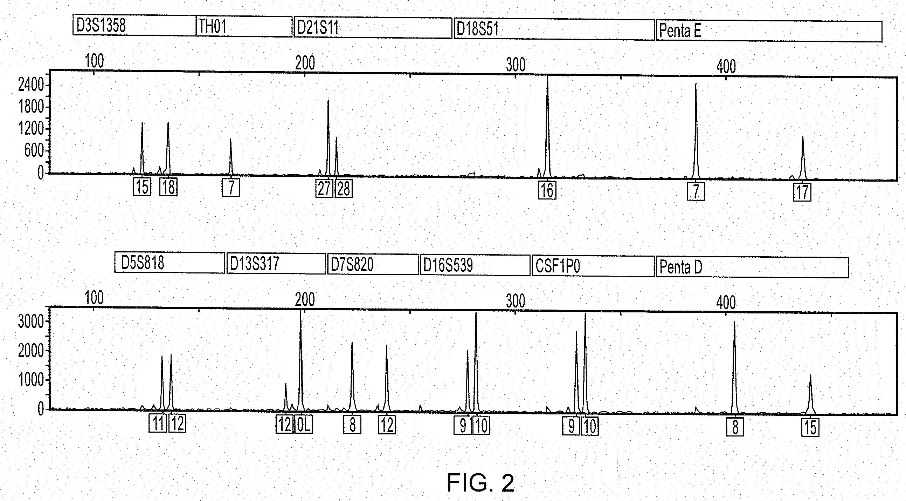

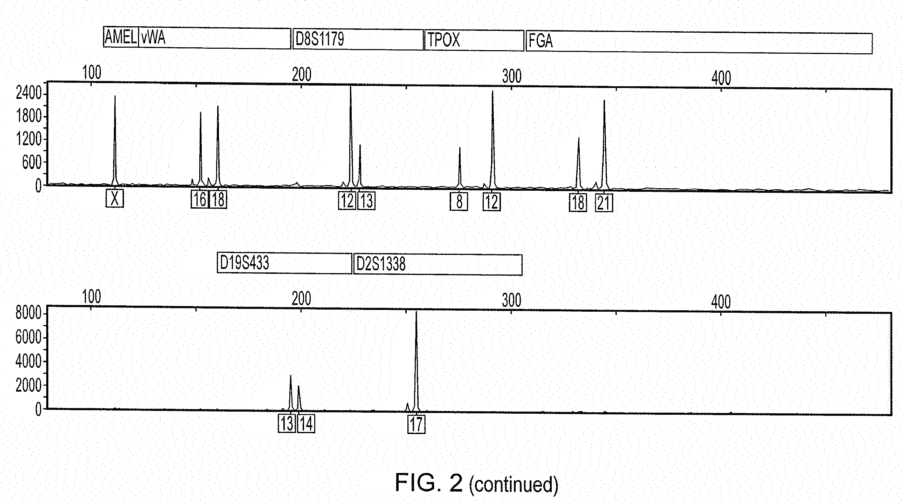

Figure 2 shows STR profile of unwashed HeLa cell spotted indicating FTA elute card combined with STR PCR reagents (replicate 2). The average peak height was 2260 RFU.

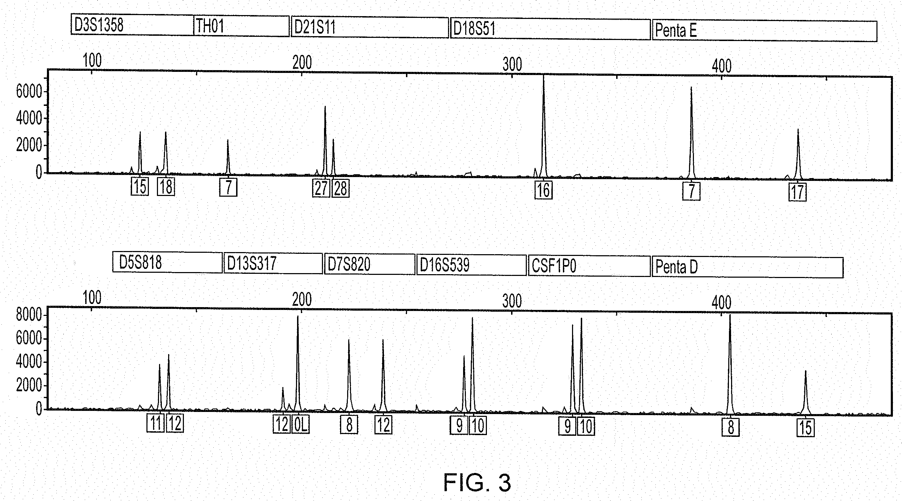

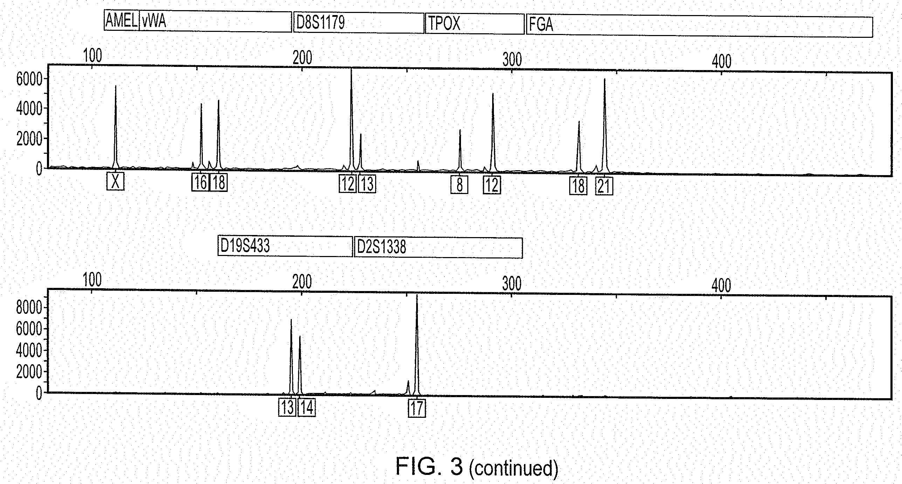

Figure 3 shows STR profile of unwashed HeLa cell spotted indicating FTA elute card combined with STR PCR reagents (replicate 3). The average peak height was 5386 RFU.

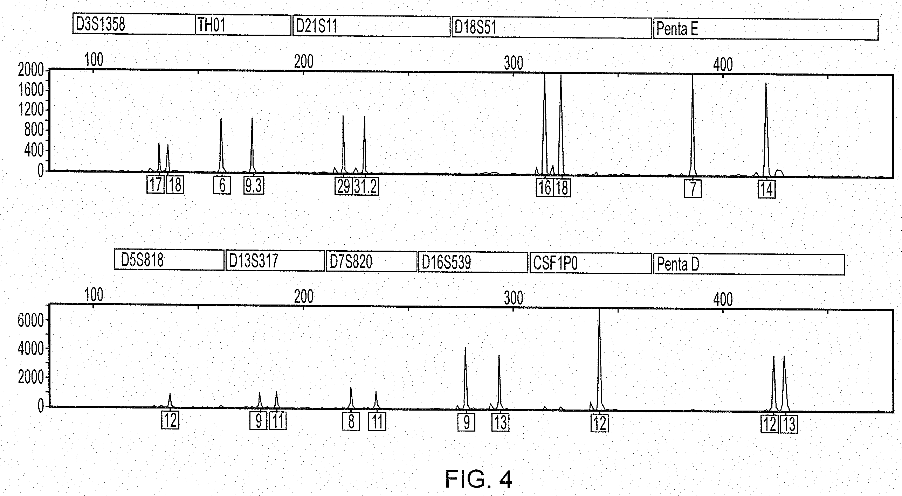

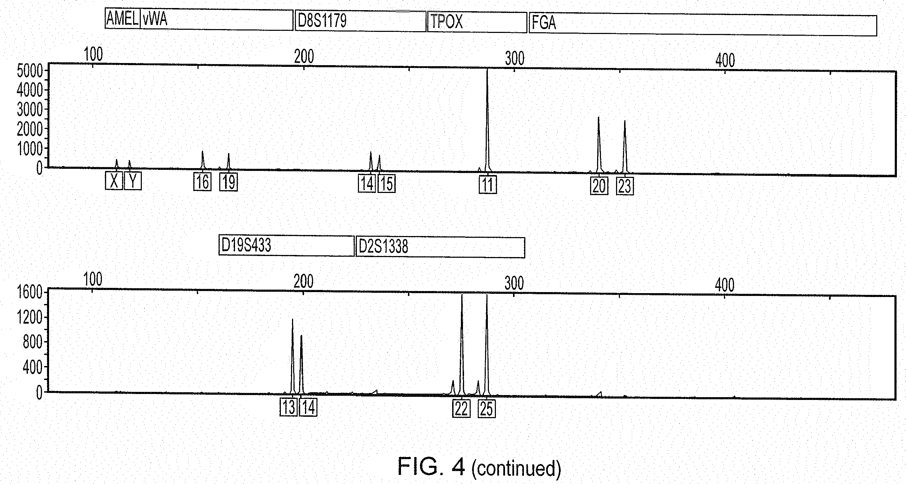

Figure 4 shows STR profile of purified genomic DNA with STR PCR reagents. The average peak height was 1904 RFU.

### Quantification of DNA from HeLa cell spotted and blood spotted FTA elute cards using gPCR (Table 5).

Cultured HeLa cells at a concentration of 1x10⁷ cell/ml or whole blood was spotted onto an indicating FTA elute card. A 3mm or 1.2mm punch was taken from the cell spotted FTA elute card and eluted using the iFTAe high throughput elution protocol or washed with 1ml of elution buffer or left unwashed. Either the sample and FTA card or 5µl of the eluate was added to the qPCR reaction containing TaqMan Rnase P detection reagents and TaqMan Universal PCR master mix. The PCR sample mix was added to individual wells of a 96 well PCR plate prior to amplification.

Indicating FTA elute high throughput elution protocol was as follows:
3mm punch was added into a 96 well PCR plate, 200µl of sterile water was added to each well, the plate was sealed and pulse vortexed three times (5 seconds each). The plate was centrifuged at 1200rpm for 2min. The water was aspirated and discarded and 60µl of sterile water was added to each well and the plate was sealed again. The plate was centrifuged at 1200rpm for 2 min and placed on a thermal cycler at 98°C for 30min.

The plate was then pulse vortexed 60 times (one pulse/sec) using a vortex mixer set on maximum speed. The plate was centrifuged at 1200rpm for 2mins and the eluate was removed from the wells using a pipette and transferred to another plate/well for quantification.

PCR reaction was set up as follows:
Standards and samples were added to the appropriate wells. The plates were sealed and centrifuged at 1000rpm for 1 minute. PCR was carried out using Applied Biosystems 7900 Real-Time PCR System under the following thermal cycling conditions:
50°C for 2min, followed by 95°C for 10min, followed by 40 cycles of: 95°C for 15sec, 60°C for 1min. The detector used was the FAM™ probe. The results are presented in Table 5.

**Table 4: Volume of reagents in the TaqMan PCR master Mix**

| **Ingredients** | **Volume** |
|---|---|
| 2X Universal Master mix | 12.5µl |
| Sterile water | 11.25µl |
| 20X RNase P primer probe | 1.25µl |
| 1.2mm or 3mm punch of FTA elute containing HeLa cells or 1.2mm or 2x3mm punch of FTA elute containing blood | 1 punch |

Table 5 shows the qPCR results of washed and unwashed blood spotted or cell spotted iFTAe card. The table shows the average yield of DNA from three qPCR reactions in ng/µl. The first 3 samples are replicates of DNA eluted from two 3mm punches of iFTAe cards and the 4^{th} sample is of a 1.2mm punch of blood spotted iFTAe card that was washed with 1ml of elution buffer and then amplified using real - time PCR (the data is the average of 3 separate samples). Samples 5 to 7 are replicates of DNA eluted from two 3mm punch of an iFTAe card spotted with HeLa cells and the 8^{th} sample is of a 3mm punch HeLa spotted iFTAe card that was washed with 1ml of elution buffer and then used in the real - time PCR machine. The last sample was of a 1.2mm punch of HeLa spotted iFTAe card that was not washed and used directly in the real - time PCR machine (the data is the average of 3 separate samples). An unspotted negative punch did not yield any detectable DNA.

**Table 5: qPCR results.**

| **Sample** | **iFTAe punch size** | **Eluted/Direct protocol** | **Average yield (ng/µl)** |
|---|---|---|---|
| Blood eluted from iFTAe Microcards (BATCH A) | 2 x 3mm punch | Eluted following iFTAe protocol | 0.039 |
| Blood eluted from iFTAe Microcards (BATCH B) | 2 x 3mm punch | Eluted following iFTAe protocol | 0.046 |
| Blood eluted from iFTAe Microcards (BATCH C) | 2 x 3mm punch | Eluted following iFTAe protocol | 0.061 |
| Blood 1.2mm from iFTAe Microcards | 1 x 1.2mm punch | Direct (washed 1ml elution buffer) | 0.039 |
| Hela cells eluted from iFTAe Microcards (BATCH A) | 1 x 3mm punch | Eluted following iFTAe protocol | 6.025 |
| Hela cells eluted from iFTAe Microcards (BATCH B) | 1 x 3mm punch | Eluted following iFTAe protocol | 5.099 |
| Hela cells eluted from iFTAe Microcards (BATCH C) | 1 x 3mm punch | Eluted following iFTAe protocol | 5.956 |
| Hela cells 3mm from iFTAe Microcards | 1 x 3mm punch | Direct (washed 1ml elution buffer) | 0.803 |
| Hela cells 1.2mm from iFTAe Microcards | 1 x 1.2mm punch | Direct (no wash) | 1.735 |

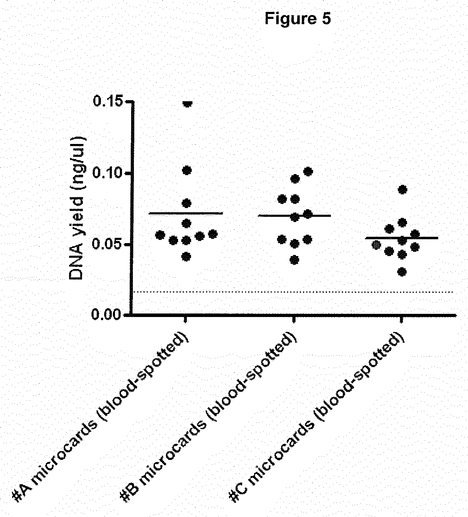

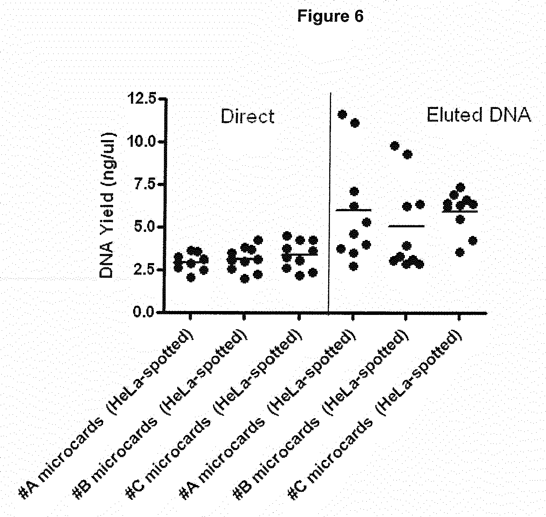

### Quantification of DNA from HeLa cell spotted and blood spotted FTA elute cards using qPCR (Figures 5 and 6)

Cultured HeLa cells at a concentration of 7.54x10⁶ cell/ml or whole blood was spotted onto an iFTAe card. A 1.2mm (HeLa and blood samples) punch was taken from the iFTAe card and washed with 1ml of sterile water, vortexed and water was removed or the sample was left unwashed. A 3mm (HeLa and blood samples) punch was taken from the iFTAe card and eluted using the iFTAe high throughput elution protocol. Either the sample spotted iFTAe card or 2 or 5µl of the eluate was added to the qPCR reaction containing TaqMan Rnase P detection reagents and TaqMan Universal PCR master mix. The PCR sample mix was added to individual wells of a 96 well PCR plate prior to amplification.

Indicating FTA elute high throughput elution protocol was as follows:
For each sample to be processed 1 x 3mm punch (HeLa sample) was placed into a 1.5ml tube. 1ml of sterile water was added to the tube and pulse vortexed three times (5 seconds each). The water was aspirated and discarded and the punches were transferred to the well of a 96 well PCR plate. 60µl of sterile water was added to each well and the plate was sealed. The plate was centrifuged at 1200rpm for 2 min and placed on a thermal cycler at 98°C for 30min. The plate was then pulse vortexed 60 times (one pulse/sec) using a vortex mixer set on maximum speed. The plate was centrifuged at 1200rpm for 2mins and the eluate was removed from the wells using a pipette and transferred to another plate/well for quantification. The plate was stored at 4ºC until quantification.

PCR reaction was set up as described above using Applied Biosystems 7900 Real-Time PCR System.

Figure 5 shows DNA yield of washed blood spotted iFTAe cards used directly in a qPCR reaction. Three different batches of iFTAe cards were used in the experiment (A, B and C).

Figure 6 shows DNA yield of unwashed HeLa cell spotted iFTAe cards either used directly in a qPCR reaction or eluated first and then used in a qPCR reaction. Three different batches of iFTAe cards were used in the experiment (A, B and C).

## Claims

1. A method for amplification of nucleic acid comprising the steps:
i) contacting a solid support comprising a chaotropic salt with a cellular sample containing nucleic acid,
ii) transferring said solid support to a reaction vessel,
iii) incubating said nucleic acid on the solid support with a nucleic acid amplification reagent solution,
iv) amplifying the nucleic acid to produce amplified nucleic acid wherein the method of amplification is a quantitative polymerase chain reaction,
v) quantifying the amplified nucleic acid and optionally,
vi) using Short Tandem Repeat (STR) profiling to produce an STR profile,
wherein steps i) to vi) are carried out in the presence of the solid support.

2. The method according to claim 1, wherein the solid support is in the reaction vessel prior to the addition of said cellular sample.

3. The method of any preceding claim, wherein the nucleic acid amplification reagent solution comprises a polymerase, deoxyribonucleotide triphosphate (dNTP), a reaction buffer and at least one primer, wherein said primer is optionally labeled with a dye.

4. The method of any preceding claim, wherein the chaotropic salt is a guanidine salt, such as a guanidine salt selected from the group consisting of guanidine thiocyanate, guanidine chloride and guanidine hydrochloride, or wherein the chaotropic salt is a sodium salt such as sodium iodide.

5. The method of any preceding claim, wherein the solid support is selected from the group consisting of a glass or silica-based solid phase medium, a plastics-based solid phase medium, a cellulose-based solid phase medium, glass fiber, glass microfiber, silica gel, silica oxide, nitrocellulose, carboxymethylcellulose, polyester, polyamide, carbohydrate polymers, polypropylene, polytetraflurorethylene, polyvinylidinefluoride, wool and porous ceramics.

6. A method for amplification of nucleic acid comprising the steps:
i) contacting a solid support comprising a chaotropic salt with a cellular sample containing nucleic acid,
ii) transferring said solid support to a reaction vessel,
iii) incubating said nucleic acid on the solid support with a nucleic acid amplification reagent solution,
iv) amplifying the nucleic acid to produce amplified nucleic acid,
v) quantifying the amplified nucleic acid wherein the amplified nucleic acid is quantified using a PCR imaging system,
wherein steps i) to v) are carried out in the presence of the solid support.

7. The method according to claim 6, wherein the solid support is in the reaction vessel prior to the addition of said cellular sample.

8. The method of claim 6 or 7, wherein the method of amplification is a polymerase chain reaction.

9. The method of claims 6 to 8, wherein said chaotropic salt is selected from the group consisting of guanidine thiocyanate, guanidine hydrochloride, guanidine chloride and sodium iodide.

10. The method of claims 6 to 9, wherein said solid support is impregnated with sodium dodecyl sulfate (SDS), ethylenediaminetetracetic acid (EDTA) and uric acid.

11. The method according to any preceding claim, wherein the cellular sample is selected from a group consisting of eukaryotic cell, prokaryotic cell, virus, bacteria, plant and tissue culture cells, and/or wherein said cellular sample is selected from the group consisting of blood, serum, semen, cerebral spinal fluid, synovial fluid, lymphatic fluid, saliva, buccal, cervical cell, vaginal cell, urine, faeces, hair, skin and muscle.

12. The method according to any preceding claim, for use as a tool selected from the group consisting of a molecular diagnostics tool, a human identification tool and a forensics tool.

13. The method according to any preceding claim, wherein the nucleic acid is stored on the solid support prior to step ii) and/or wherein the nucleic acid is stored on the solid support for at least 30 minutes.

## Patentansprüche

1. Verfahren zur Amplifikation von Nukleinsäure, umfassend die Schritte:
i) Inkontaktbringen eines festen Trägers, der ein chaotropes Salz umfasst, mit einer Zellprobe, die Nukleinsäure enthält,
ii) Überführen des festen Trägers in ein Reaktionsgefäß,
iii) Inkubieren der Nukleinsäure auf dem festen Träger mit einer Nukleinsäuren-Amplifikationsreagenzlösung,
iv) Amplifizieren der Nukleinsäure zur Herstellung amplifizierter Nukleinsäure, wobei das Amplifikationsverfahren eine quantitative Polymerasekettenreaktion ist,
v) Quantifizierung der amplifizierten Nukleinsäure und optional
vi) Verwendung einer Short Tandem Repeat (STR)-Profilierung zur Herstellung eines STR-Profils,
wobei die Schritte i) bis vi) in Gegenwart des festen Trägers durchgeführt werden.

2. Verfahren nach Anspruch 1, wobei sich der feste Träger vor der Zugabe der Zellprobe im Reaktionsgefäß befindet.

3. Verfahren nach einem der vorstehenden Ansprüche, wobei die Nukleinsäuren-Amplifikationsreagenzlösung eine Polymerase, Desoxyribonukleotidtriphosphat (dNTP), einen Reaktionspuffer und zumindest einen Primer umfasst, wobei der Primer optional mit einem Farbstoff gekennzeichnet ist.

4. Verfahren nach einem der vorstehenden Ansprüche, wobei das chaotrope Salz ein Guanidinsalz wie ein Guanidinsalz ist, ausgewählt aus der Gruppe bestehend aus Guanidinthiocyanat, Guanidinchlorid und Guanidinhydrochlorid, oder wobei das chaotrope Salz ein Natriumsalz wie Natriumiodid ist.

5. Verfahren nach einem der vorstehenden Ansprüche, wobei der feste Träger ausgewählt ist aus der Gruppe bestehend aus einem Festphasenmedium auf Glas- oder Kieselsäurebasis, einem Festphasenmedium auf Kunststoffbasis, einem Festphasenmedium auf Zellulosebasis, Glasfaser, Glasmikrofaser, Kieselgel, Kieselsäureoxid, Nitrocellulose, Carboxymethylcellulose, Polyester, Polyamid, Kohlenhydratpolymeren, Polypropylen, Polytetrafluorethylen, Polyvinylidinefluorid, Wolle und poröser Keramik.

6. Verfahren zur Amplifikation von Nukleinsäure, umfassend die Schritte:
i) Inkontaktbringen eines festen Trägermaterials, das ein chaotropes Salz umfasst, mit einer Zellprobe, die Nukleinsäure enthält,
ii) Überführen des festen Trägermaterials in ein Reaktionsgefäß,
iii) Inkubieren der Nukleinsäure auf dem festen Trägermaterial mit einer Nukleinsäuren-Amplifikationsreagenzlösung,
iv) Amplifizieren der Nukleinsäure zur Herstellung von amplifizierter Nukleinsäure,
v) Quantifizierung der amplifizierten Nukleinsäure, wobei die amplifizierte Nukleinsäure mittels eines PCR-Bildgebungssystems quantifiziert wird,
wobei die Schritte i) bis vi) in Gegenwart des festen Trägers durchgeführt werden.

7. Verfahren nach Anspruch 6, wobei sich das feste Trägermaterial vor der Zugabe der Zellprobe im Reaktionsgefäß befindet.

8. Verfahren nach Anspruch 6 oder 7, wobei das Verfahren der Amplifikation eine Polymerase-Kettenreaktion ist.

9. Verfahren nach Anspruch 6 bis 8, wobei das chaotrope Salz ausgewählt ist aus der Gruppe bestehend aus Guanidinthiocyanat, Guanidinhydrochlorid, Guanidinchlorid und Natriumiodid.

10. Verfahren nach Anspruch 6 bis 9, wobei der feste Träger mit Natriumdodecylsulfat (SDS), Ethylendiamintetracetic Säure (EDTA) und Harnsäure imprägniert wird.

11. Verfahren nach einem der vorstehenden Ansprüche, wobei die Zellprobe aus einer Gruppe ausgewählt ist, die aus eukaryontischen Zellen, prokaryontischen Zellen, Viren, Bakterien, Pflanzen- und Gewebekulturzellen besteht, und/oder wobei die Zellprobe aus der Gruppe ausgewählt ist, die aus Blut, Serum, Samen, zerebraler Rückenmarksflüssigkeit, Gelenkflüssigkeit, Lymphflüssigkeit, Speichel, bukkalen, zervikalen Zellen, Vaginalzelle, Urin, Kot, Haar, Haut und Muskel besteht.

12. Verfahren nach einem der vorstehenden Ansprüche zur Verwendung als Werkzeug, ausgewählt aus der Gruppe bestehend aus einem molekulardiagnostischen Werkzeug, einem Werkzeug zur Identifizierung von Menschen und einem forensischen Werkzeug.

13. Verfahren nach einem der vorstehenden Ansprüche, wobei die Nukleinsäure vor Schritt ii) auf dem festen Träger gelagert wird und/oder wobei die Nukleinsäure auf dem festen Träger über einen Zeitraum von mindestens 30 Minuten gelagert wird.

## Revendications

1. Procédé pour l'amplification d'acide nucléique comprenant les étapes :
i) mise en contact d'un support solide comprenant un sel chaotropique avec un échantillon cellulaire contenant un acide nucléique,
ii) transfert dudit support solide jusqu'à un récipient de réaction,
iii) incubation dudit acide nucléique sur le support solide avec une solution de réactif d'amplification d'acide nucléique,
iv) amplification de l'acide nucléique pour produire un acide nucléique amplifié dans lequel le procédé d'amplification est une amplification en chaîne par polymérase quantitative,
v) détermination de la quantité de l'acide nucléique amplifié et de manière facultative,
vi) utilisation d'un profilage de brève séquence répétée en tandem (STR) pour produire un profil STR,
dans lequel les étapes i) à vi) sont effectuées en présence du support solide.

2. Procédé selon la revendication 1, dans lequel le support solide est dans le récipient de réaction avant l'ajout dudit échantillon cellulaire.

3. Procédé selon l'une quelconque des revendications précédentes, dans lequel la solution de réactif d'amplification d'acide nucléique comprend une polymérase, du triphosphate de désoxyribonucléotide (dNTP), un tampon de réaction et au moins une amorce, dans lequel ladite amorce est marquée de manière facultative avec un colorant.

4. Procédé selon l'une quelconque des revendications précédentes, dans lequel le sel chaotropique est un sel de guanidine, tel qu'un sel de guanidine sélectionné à partir du groupe constitué par du thiocyanate de guanidine, du chlorure de guanidine et du chlorhydrate de guanidine, ou dans lequel le sel chaotropique est un sel de sodium comme de l'iodure de sodium.

5. Procédé selon l'une quelconque des revendications précédentes, dans lequel le support solide est sélectionné à partir du groupe constitué par un verre ou un milieu en phase solide à base de silice, un milieu en phase solide à base de plastique, un milieu en phase solide à base de cellulose, de la fibre de verre, de la microfibre de verre, du gel de silice, de l'oxyde de silice, de la nitrocellulose, de la carboxyméthylcellulose, du polyester, du polyamide, des polymères d'hydrate de carbone, du polypropylène, du polytétrafluroréthylène, du polyvinylidinefluorure, de la laine et de la céramique poreuse.

6. Procédé pour l'amplification d'acide nucléique comprenant les étapes de :
i) mise en contact d'un support solide comprenant un sel chaotropique avec un échantillon cellulaire contenant un acide nucléique,
ii) transfert dudit support solide jusqu'à un récipient de réaction,
iii) incubation dudit acide nucléique sur le support solide avec une solution de réactif d'amplification d'acide nucléique,
iv) amplification de l'acide nucléique pour produire un acide nucléique amplifié,
v) détermination de la quantité de l'acide nucléique amplifié dans laquelle l'acide nucléique amplifié est évalué quantitativement en utilisant un système d'imagerie PCR,
dans lequel les étapes i) à v) sont effectuées en présence du support solide.

7. Procédé selon la revendication 6, dans lequel le support solide est dans le récipient de réaction avant l'ajout dudit échantillon cellulaire.

8. Procédé selon la revendication 6 ou 7, dans lequel le procédé d'amplification est une amplification en chaîne par polymérase.

9. Procédé selon les revendications 6 à 8, dans lequel ledit sel chaotropique est sélectionné à partir du groupe constitué de thiocyanate de guanidine, de chlorhydrate de guanidine, de chlorure de guanidine et d'iodure de sodium.

10. Procédé selon les revendications 6 à 9, dans lequel ledit support solide est imprégné de sulfate de dodécyle sodique (SDS), d'acide éthylène-diamine-tétracétique (EDTA) et d'acide urique.

11. Procédé selon l'une quelconque des revendications précédentes, dans lequel l'échantillon cellulaire est sélectionné à partir d'un groupe constitué de cellules eucaryotes, de cellules procaryotes, de virus, de bactéries, de cultures de cellules tissulaires et végétales, et/ou dans lequel ledit échantillon cellulaire est sélectionné à partir du groupe constitué par du sang, du sérum, du sperme, du liquide céphalorachidien, du liquide synovial, du liquide lymphatique, de la salive, des cellules buccales, des cellules cervicales, des cellules vaginales, de l'urine, des fèces, des cheveux, de la peau et du muscle.

12. Procédé selon l'une quelconque des revendications précédentes, pour une utilisation en tant qu'outil sélectionné à partir du groupe constitué par un outil de diagnostic moléculaire, un outil d'identification humaine et un outil criminalistique.

13. Procédé selon l'une quelconque des revendications précédentes, dans lequel l'acide nucléique est stocké sur le support solide avant l'étape ii) et/ou dans lequel l'acide nucléique est stocké sur le support solide pendant au moins 30 minutes.
